# EUROPEAN PATENT APPLICATION

(11) **EP 3 756 536 A1**
(43) Date of publication of application: **30.12.2020**
(21) Application number: 19183063.7
(22) Date of filing: 27.06.2019
(51) Int. Cl.: A61B 5/00, A61B 5/05, A61B 5/055, A61B 5/113, A61B 5/11, A61B 6/00

(54) **MEDICAL IMAGING SYSTEM**

(71) Applicant: Koninklijke Philips N.V., 5656 AG Eindhoven (NL)
(72) Inventor: Wirtz, Daniel, 5656 AE Eindhoven (NL); Leussler, Christoph, 5656 AE Eindhoven (NL); Vogtmeier, Gereon, 5656 AE Eindhoven (NL); Vuppala, Sunil Kumar, 5656 AE Eindhoven (NL)
(74) Representative: de Haan, Poul Erik

(57) **Abstract**

The present invention relates to a medical imaging system (10), comprising an image acquisition unit (20), a radar apparatus (30), and a processing unit (50). Te image acquisition unit is configured to acquire medical image data of a patient when positioned for medical image acquisition. The radar apparatus comprises a transmitter (60) and receiver (70). The radar apparatus is configured to operate over radio-wave and/or microwave and/or terahertz radiation frequencies to acquire depth information relating to the patient when positioned for medical image acquisition. The processing unit is configured to determine at least one operational parameter for the radar apparatus comprising utilization of data acquired by the imaging system. The processing unit is configured to operate the radar apparatus to acquire operational depth information relating to the patient comprising utilization of the at least one operational parameter. The processing unit is configured to control the image acquisition unit comprising utilization of the operational depth information.

## Description

### FIELD OF THE INVENTION

The present invention relates to a medical imaging system, a method of medical imaging, as well as to a computer program element and a computer readable medium.

### BACKGROUND OF THE INVENTION

When acquiring medical imagery with for example a Magnetic Resonance imaging MRI system or a Computer tomography CT X-ray system, before actually acquiring imagery (or scanning), a number of input parameters need to be provided to the systems in order to ensure proper scan preparation. Depending on body size, body weight, patient position and anatomy to be scanned, a protocol is chosen and modified to fit the patient. Typically, these data have to be entered manually. Physiology parameters (necessary for triggering scans) have to be measured using dedicated sensors.

Thus, when imaging certain parts of the body, imagery is acquired at a certain breathing state or at a certain state during the heart-cycle is used. This is true especially for abdominal and cardiac imaging, since motion would otherwise blur the overall image (combined of several single imaging shots). According, high-quality signals for triggering the imaging machine are required. For breathing motion detection this is typically done using a pneumatic bellows that is tied to the patient and translates breathing motion in pressure changes that reflect the breathing curve over time. Alternatively, analysis of a video stream can be used for the same purpose. In cardiac imaging a full-blown ECG or pulse-detection using a finger clip sensor measuring the pulsation are common.

However, for detecting breathing motion, the bellows used are rather prone to misplacement and often provide only a poor quality signal. Different types of breathing patterns (for example chest-breathing vs. stomach-breathing) are not automatically detected, and all this has to be taken into consideration before placing the device.

Regarding camera based breathing signal detection, this can be very sensitive to small motions and can detect different breathing patterns. However, for this technique an unobstructed line of sight to the abdominal and/or chest region of the patient is mandatory. In clinical settings this requirement is not always easy to realize.

For cardiac motion detection, often real-time information is required (especially in case of arrhythmic patients). Both, photoplethysmogram (PPG) using a pulse oximeter frequently worn on the finger using a fingerclip and camera imagery (using for example a colour blush of the facial skin as a diagnostic) based methods come with physiological delays. Thus, the PPG signal taken at the fingertip and the facial blush information, useable to determine an image acquisition trigger point, are delayed by up to several 100ms compared to the actual heartbeat. This renders triggering the scan acquisition on those signals suboptimal at best.

There is a need to address these issues.

### SUMMARY OF THE INVENTION

It would be advantageous to have improved means of determining trigger information for the acquisition of medical imagery with a medical image acquisition unit. The object of the present invention is solved with the subject matter of the independent claims, wherein further embodiments are incorporated in the dependent claims. It should be noted that the following described aspects and examples of the invention apply also to the medical imaging system, the method of medical imaging, as well as to the computer program element and a computer readable medium.

In a first aspect, there is provided a medical imaging system, comprising:
- an image acquisition unit;
- a radar apparatus; and
- a processing unit.

The image acquisition unit is configured to acquire medical image data of a patient when positioned for medical image acquisition. The radar apparatus comprises a transmitter and receiver. The radar apparatus is configured to operate over radio-wave and/or microwave and/or terahertz radiation frequencies to acquire depth information relating to the patient when positioned for medical image acquisition. The processing unit is configured to determine at least one operational parameter for the radar apparatus comprising utilization of data acquired by the imaging system. The processing unit is configured to operate the radar apparatus to acquire operational depth information relating to the patient comprising utilization of the at least one operational parameter. The processing unit is configured to control the image acquisition unit comprising utilization of the operational depth information.

In an example, the processing unit is configured to operate the radar apparatus to acquire preliminary depth information relating to the patient when positioned for medical image acquisition. Determination of the at least one operational parameter can then comprise utilization of the preliminary depth information.

In other words, a radar based depth sensitive sensor apparatus, the wavelength frequencies of which can be determined by the radar apparatus itself, is used within an image acquisition unit in order to perform for example selective motion and/or physiological triggering for imaging by the image acquisition unit. To put this another way, the optimum radar frequency can be determined by the system itself (e.g. from a best signal-to-noise of the physiology signal).

In an example, the system comprises at least one camera configured to acquire optical image data of the patient when positioned for medical image acquisition. Determination of the at least one operational parameter can then comprise utilization of the optical image data of the patient when positioned for medical image acquisition.

In other words, a depth sensitive sensor apparatus, the wavelength frequencies of which can be determined from camera imagery on the basis of for example the size, position, and pose of the patient, is used within an image acquisition unit in order to perform for example selective motion and/or physiological triggering for imaging by the image acquisition unit.

This determination of the wavelength frequencies of the radar apparatus can also utilize radar data itself, for example signal to noise of the physiology signal, where this is augmented by image data providing information such as size, position and pose of the patient for example.

In an example, the at least one operational parameter comprises an operational radiation frequency for the radar apparatus. The processing unit is configured to control the radar apparatus to operate at the operational radiation frequency to acquire the operational depth information relating to the patient.

In this manner, if for example a patient's heart is being imaged by the image acquisition unit, radar data and/or optical data can determine how large the subject is, from which a determination can be made of what low frequency radio waves are optimum to scan inside the patient by the radar apparatus to provide depth information from which movement of the heart can be determined. Then from this depth information, the image acquisition unit can be triggered to acquire a series of medical image data at exactly the same time point in the heart's beat cycle.

In an example, the at least one operational parameter comprises an indication to scan at least one specific region relative to a central axis of the radar apparatus. The processing unit is configured to control the radar apparatus to scan the at least one specific region to acquire the operational depth information relating to the patient.

Thus, for example if it was required to acquire medical image data of the chest of a subject, then the radar data and/or the optical image data can be used to determine exactly where the subject's chest is and how large the subject's chest is. This information is used to configure the radar apparatus to operate at an optimum radiation frequency range to probe this region and the radar apparatus can probe only that region as required. Then, for example if medical imagery at a specific point in the patient's breathing cycle is required, then from the operational depth information acquired by the radar apparatus the image acquisition unit can be triggered to acquire images periodically at exactly the required point in time in the patient's breathing cycle.

In an example, the transmitter of the radar apparatus comprises a plurality of transmitter elements.

In this manner, different parts of the patient can examined individually more efficiently.

In an example, the transmitter comprises a phased array transmitter. The processing unit is configured to control the radar apparatus to vary a direction of radiation with respect to the central axis.

In this manner, the radar apparatus can operate in a beam steering mode to accurately probe the required body part and/or region of the patient.

In an example, the processing unit is configured to utilize the operational depth information to trigger the image acquisition unit to acquire the medical image data.

In an example, the transmitter and receiver of the radar apparatus are located on the same side of the patient when positioned for medical image acquisition.

In this manner, reflection and/or scatter based depth information is provided from the patient. Thus, for example a transmitter and receiver, which can be in the form of a transceiver, can be placed on one side of where the patient is positioned. If the wavelength range of the emitted radiation is such to reflect from the patient, either from their clothing or propagating through their clothing and reflecting from their body, then knowledge of the time of flight to and from the patient provides information on their size. This is because it is known how large in depth the area is within which the patient is positioned. Also as the patient moves, through for example breathing or the heart beating, then change in distance to the patient provides information on this movement of the patient. Similarly, low wavelength radio waves can be generated that for example can propagate all the way through the patient, reflect on the other side of the patient and propagate back through the patient, will suffer time delay that depend on the depth and material type of the patient, and this can be used to provide size, position, and movement information of the patient. Some radiation will also back-reflect and scatter back towards the receiver on its way through the patient, thereby probing the internal parts of the patient, enabling information on for example movement of the heart, diaphragm and other internal parts of the patient to be determined.

In an example, the transmitter and receiver of the radar apparatus are located on opposite sides of the patient when positioned for medical image acquisition.

In other words, low wavelength radio waves, for example can be generated and where some of that radiation propagates all the way through the patient, and different paths suffer different time delays that depend on the depth and material type of the patient, and this can be used to provide size, position, and movement information of the patient. This enables information on for example movement of the heart, diaphragm and other internal parts of the patient to be determined.

In an example, the system comprises at least one radiation reflector.

In this manner, the signal to noise can be increased, Thus, radiation emitted by the radar apparatus can reflect from the front surface of the patient and provide for an increased signal, and indeed appropriate wavelengths, such as low wavelength radio waves emitted by the radar apparatus that pass through the patient can then be reflected from the "back" surface of the patient and pass back through the patient to provide a greater signal to noise and improved information relating to this outer part of the patient.

In an example, the radiation reflector associated with the part of the image acquisition unit is comprised within or on a mattress of the image acquisition unit.

This provides for convenient positioning of the reflectors, with no extra items required to be placed upon the patient for example.

In an example, the image acquisition unit is a Magnetic Resonance image acquisition unit, and wherein one or more radiation reflectors are comprised within one or more RF coil radiation receivers of the image acquisition unit.

In a second aspect, there is provided a method of medical imaging with a medical imaging system, comprising:
a) positioning a patient at least partially within an image acquisition unit of the medical imaging system;
b) acquiring by the medical imaging system data of the patient when positioned at least partially within the image acquisition unit;
c) determining by a processing unit of the medical imaging system at least one operational parameter for a radar apparatus of the medical imaging system comprising utilizing the optical image data, wherein the radar apparatus comprises a transmitter and receiver;
d) operating by the processing unit the radar apparatus to acquire operational depth information relating to the patient when positioned at least partially within an image acquisition unit comprising utilizing the at least one operational parameter; and
e) controlling by the processing unit the image acquisition unit to acquire medical image data of the patient comprising utilizing the operational depth information.

According to another aspect, there is provided a computer program element controlling one or more of the apparatuses/systems as previously described which, if the computer program element is executed by a processing unit, is adapted to perform one or more of the methods as previously described.

According to another aspect, there is provided a computer readable medium having stored computer element as previously described.

The computer program element can for example be a software program but can also be a FPGA, a PLD or any other appropriate digital means.

Advantageously, the benefits provided by any of the above aspects equally apply to all of the other aspects and vice versa.

The above aspects and examples will become apparent from and be elucidated with reference to the embodiments described hereinafter.

### BRIEF DESCRIPTION OF THE DRAWINGS

Exemplary embodiments will be described in the following with reference to the following drawings:
Fig. 1 shows a schematic set up of an example of a medical imaging system;
Fig. 2 shows a method of medical imaging;
Fig. 3 shows a detailed representation of an example of a medical imaging system; and
Fig. 4 shows an example of an RF coil element, and an example of a coil array built up from a number of such RF coil elements.

### DETAILED DESCRIPTION OF THE EMBODIMENTS

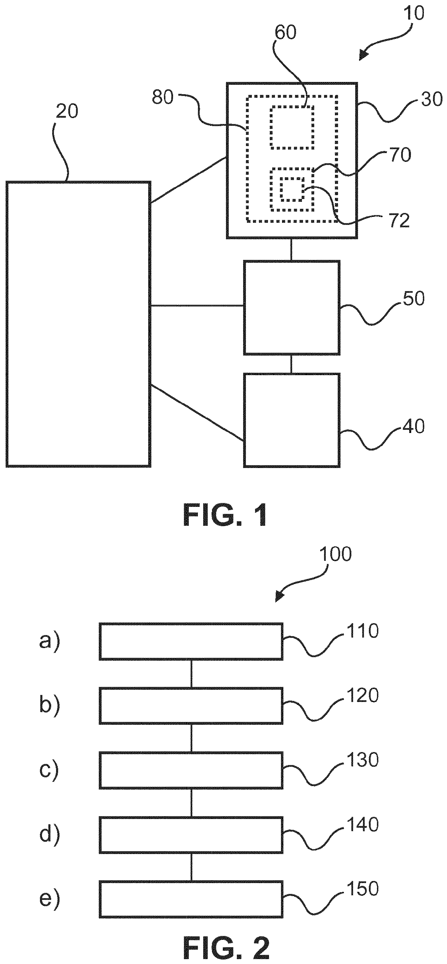

Fig. 1 shows an example of a medical imaging system 10, where essential elements are shown in solid lines, and optional elements are shown in dashed lines. The system 10 comprises an image acquisition unit 20, a radar apparatus 30, and a processing unit 50. The image acquisition unit is configured to acquire medical image data of a patient when positioned for medical image acquisition. The radar apparatus comprises a transmitter 60 and receiver 70. The radar apparatus is configured to operate over radio-wave and/or microwave and/or terahertz radiation frequencies to acquire depth information relating to the patient when positioned for medical image acquisition. The processing unit is configured to determine at least one operational parameter for the radar apparatus comprising utilization of data acquired by the imaging system. The processing unit is configured also to operate the radar apparatus to acquire operational depth information relating to the patient comprising utilization of the at least one operational parameter. The processing unit is configured also to control the image acquisition unit comprising utilization of the operational depth information.

In an example, the depth information acquiring by the radar apparatus is converted into motion information. Thus, configuration of the radar apparatus to acquire operational depth information can be based on motion information derived from depth information. Also control of the image acquisition unit can be based on operational motion information derived from operational depth information.

In an example, the transmitter 60 and receiver 70 are combined within a transceiver 80.

In an example, the processing unit is configured to implement a machine learning algorithm to process the operational depth information to control the image acquisition unit.

In an example, the processing unit is configured to implement a recurrent neural network to process the operational depth information to control the image acquisition unit.

According to an example, the processing unit is configured to operate the radar apparatus to acquire preliminary depth information relating to the patient when positioned for medical image acquisition. Determination of the at least one operational parameter can then comprise utilization of the preliminary depth information.

In an example, the processing unit is configured to implement a machine learning algorithm to process the preliminary depth information to determine the at least one operational parameter.

In an example, the processing unit is configured to implement a convolutional neural network to process the preliminary depth information to determine the at least one operational parameter.

In an example, the processing unit is configured to implement a capsule network to process the preliminary depth information to determine the at least one operational parameter.

According to an example, the system comprises at least one camera 40 configured to acquire optical image data of the patient when positioned for medical image acquisition. Determination of the at least one operational parameter can then comprise utilization of the optical image data of the patient when positioned for medical image acquisition.

In an example, the at least one camera operates over visible wavelengths.

In an example, the at least one camera operates over infra-red wavelengths.

In an example, the processing unit is configured to implement a machine learning algorithm to process the optical image data to determine the at least one operational parameter.

In an example, the processing unit is configured to implement a convolutional neural network to process the optical image data to determine the at least one operational parameter.

In an example, the processing unit is configured to implement a capsule network to process the optical image data to determine the at least one operational parameter.

In an example, the processing unit is configured to implement a machine learning algorithm to process the preliminary depth information and the optical image data to determine the at least one operational parameter.

In an example, the processing unit is configured to implement a convolutional neural network to process the preliminary depth information the optical image data to determine the at least one operational parameter.

In an example, the processing unit is configured to implement a capsule network to process the preliminary depth information the optical image data to determine the at least one operational parameter

Thus optical imagery can be utilized to determine what the best radar based interrogation wavelengths should be selected, where that selection can be based on the size, orientation, and position of the patient and on the body part to be imaged by the medical image acquisition unit, such as a CTI, or MRI scanner. Radar information itself can be used in combination with this optical image data, where for example signal to noise information from the radar data can be utilized with the image data to determine the optimum operational frequencies of the radar apparatus to determine depth and/or motion data to provide for optimum triggering of a medical image acquisition unit.

Expert knowledge and testing can be used to determine the best interrogating radar wavelengths for a particular situation, where there is then also acquired associated camera imagery. This is used to train a neural network, that can then be used to determine the radar apparatus setup from newly acquired camera imagery. Furthermore, the radar data can be rather complex. For example, depending upon the wavelength range going from radio waves, terahertz to low radio waves, radiation can reflect from the patient and their clothing, pass through their clothing and reflect from the outer part of their body or pass through at least some of their body, reflecting, scattering and suffering time delay as it does so. However, a neural network can be trained where for example a known size, position, and movement of a patient in terms of for movement phase of the heart and breathing is used as ground truth information with the associated radar based training data in order to determine how that radar data can be used to trigger a medical image acquisition system at the same point in for example the movement cycle of the heart or breathing cycle. Then, real radar data can be analysed, knowing the operational parameters of the radar apparatus in terms of its operating wavelength, and size and position of the patient being interrogated, determined from camera imagery, enables the returning radar signal to be used as an effective trigger signal to trigger when the medical image acquisition unit is to acquire its imagery.

According to an example, the at least one operational parameter comprises an operational radiation frequency for the radar apparatus. The processing unit is configured to control the radar apparatus to operate at the operational radiation frequency to acquire the operational depth information relating to the patient.

According to an example, the at least one operational parameter comprises an indication to scan at least one specific region relative to a central axis of the radar apparatus. The processing unit is configured to control the radar apparatus to scan the at least one specific region to acquire the operational depth information relating to the patient.

According to an example, the transmitter of the radar apparatus comprises a plurality of transmitter elements.

According to an example, the transmitter comprises a phased array transmitter. The processing unit is configured to control the radar apparatus to vary a direction of radiation with respect to the central axis.

According to an example, the processing unit is configured to utilize the operational depth information to trigger the image acquisition unit to acquire the medical image data.

According to an example, the transmitter and receiver of the radar apparatus are located on the same side of the patient when positioned for medical image acquisition.

According to an example, the transmitter and receiver of the radar apparatus are located on opposite sides of the patient when positioned for medical image acquisition.

According to an example, the system comprises at least one radiation reflector.

In an example, the at least one radiation reflector comprises a radiation reflector associated with a part of the image acquisition unit adjacent to which at least a part of the patient is situated when positioned for medical image acquisition.

Thus, the patient can lie on this reflector or stand up against it for example, or the reflector can be placed on top of the patient who is lying on the scanner bed.

This reflector can also be integrated into a Magnetic Resonance (MR) surface coil. In this case the radar transmitter and receiver would be placed on the other side of the patient to the reflector. Thus, the reflector can be above the patient and the radar transmitter and receiver placed below the patient, or the radar transmitter and receiver can be above the patient and the reflector below the patient, and even integrated into an MR table.

In an example, each radiation reflector is comprised from a plurality of reflector elements. In this manner, in an MR imaging unit, the generation of eddy current during the MR excitation pulse is mitigated

According to an example, the radiation reflector associated with the part of the image acquisition unit is comprised within or on a mattress of the image acquisition unit.

In an example, the at least one radiation reflector comprises a radiation reflector configured to be applied to the outside of the patient.

In an example, the radiation reflector configured to be applied to the outside of the patient is a sticker.

According to an example, the image acquisition unit is a Magnetic Resonance image acquisition unit. One or more radiation reflectors can be comprised within one or more RF coil radiation receivers 72 of the image acquisition unit.

In an example, the processing unit is configured to turn the one or more RF coil radiation receivers on and off.

Thus, highly reflective reflectors can be provided for a MRI system that make use of RF coils already used for MRI signal detection.

Fig. 2 shows a method 100 of medical imaging with a medical imaging system in its basic steps. The method 100 comprises:
in a positioning step 110, also referred to as step a), positioning a patient at least partially within an image acquisition unit of the medical imaging system;
in an acquiring step 120, also referred to as step b), acquiring by the medical imaging system data of the patient when positioned at least partially within the image acquisition unit;
in a determining step 130, also referred to as step c), determining by a processing unit of the medical imaging system at least one operational parameter for a radar apparatus of the medical imaging system comprising utilizing the optical image data, wherein the radar apparatus comprises a transmitter and receiver;
in an operating step 140, also referred to as step d), operating by the processing unit the radar apparatus to acquire operational depth information relating to the patient when positioned at least partially within an image acquisition unit comprising utilizing the at least one operational parameter; and
in a controlling step 150, also referred to as step e), controlling by the processing unit the image acquisition unit to acquire medical image data of the patient comprising utilizing the operational depth information.

It is to be noted that method steps can run in parallel, this medical imaging and radar-based motion deduction would can run in parallel, where the radar apparatus triggers the imaging in a continuously repeating manner. To put this another way, the radar apparatus can permanently provide a physiological signal curve e.g. breathing or (ECG-like) cardiac motion, even without the imaging part. However, as soon as triggered imaging is required, the trigger can then be deduced from the radar apparatus output curve.

In an example, the transmitter and receiver are combined within a transceiver. In an example, step b) comprises operating by the processing unit the radar apparatus to acquire preliminary depth information relating to the patient when positioned for medical image acquisition, and wherein step c) comprises utilization of the preliminary depth information.

In an example, step c) comprises implementing by the processing unit a machine learning algorithm to process the preliminary depth information to determine the at least one operational parameter.

In an example, step c) comprises implementing by the processing unit a convolutional neural network to process the preliminary depth information to determine the at least one operational parameter.

In an example, step c) comprises implementing by the processing unit a capsule network to process the preliminary depth information to determine the at least one operational parameter.

In an example, step b) comprises acquiring by at least one camera of the medical imaging system optical image data of the patient when positioned for medical image acquisition, and wherein step c) comprises utilization of the optical image data of the patient when positioned for medical image acquisition.

In an example, step c) comprises implementing by the processing unit a machine learning algorithm to process the optical image data to determine the at least one operational parameter.

In an example, step c) comprises implementing by the processing unit a convolutional neural network to process the optical image data to determine the at least one operational parameter.

In an example, step c) comprises implementing by the processing unit a capsule network to process the optical image data to determine the at least one operational parameter.

In an example, step c) comprises implementing by the processing unit a machine learning algorithm to process the preliminary depth information and the optical image data to determine the at least one operational parameter.

In an example, step c) comprises implementing by the processing unit a convolutional neural network to process the preliminary depth information the optical image data to determine the at least one operational parameter.

In an example, step c) comprises implementing by the processing unit a capsule network to process the preliminary depth information the optical image data to determine the at least one operational parameter

In an example, step e) comprises implementing by the processing unit a machine learning algorithm to process the operational depth information to control the image acquisition unit.

In an example, step e) comprises implementing by the processing unit a recurrent neural network to process the operational depth information to control the image acquisition unit.

In an example, in step c) the determined at least one operational parameter comprises an operational radiation frequency for the radar apparatus, and wherein step d) comprises controlling by the processing unit the radar apparatus to operate at the operational radiation frequency to acquire the operational depth information relating to the patient.

In an example, in step c) the determined at least one operational parameter comprises an indication to scan at least one specific region relative to a central axis of the radar apparatus, and wherein step d) comprises controlling by the processing unit the radar apparatus to scan the at least one specific region to acquire the operational depth information relating to the patient.

In an example, the radar apparatus comprises a transmitter comprising a plurality of transmitter elements.

In an example, the radar apparatus comprises a receiver comprising a plurality of receiver elements.

In an example, the transmitter comprises a phased array transmitter, and wherein step d) comprises controlling by the processing unit the radar apparatus to vary a direction of radiation with respect to the central axis.

In an example, step e) comprises utilizing by the processing unit the operational depth information to trigger the image acquisition unit to acquire the medical image data.

In an example, the transmitter and receiver of the radar apparatus are located on the same side of the patient when positioned for medical image acquisition.

In an example, the transmitter and receiver of the radar apparatus are located on opposite sides of the patient when positioned for medical image acquisition.

In an example, method comprises positioning at least one radiation reflector relative to the patient and the radar apparatus.

In an example, the at least one radiation reflector comprises a radiation reflector associated with a part of the image acquisition unit adjacent to which at least a part of the patient is situated.

In an example, each radiation reflector is comprised from a plurality of reflector elements.

In an example, the radiation reflector associated with the part of the image acquisition unit is comprised within or on a mattress of the image acquisition unit. In an example, the method comprises applying a radiation reflector to the outside of the patient.

In an example, the radiation reflector applied to the outside of the patient is a sticker.

In an example, the image acquisition unit is a Magnetic Resonance image acquisition unit, and wherein one or more radiation reflectors are comprised within one or more RF coil radiation receivers of the image acquisition unit.

In an example, the processing unit is configured to turn the one or more RF coil radiation receivers on and off.

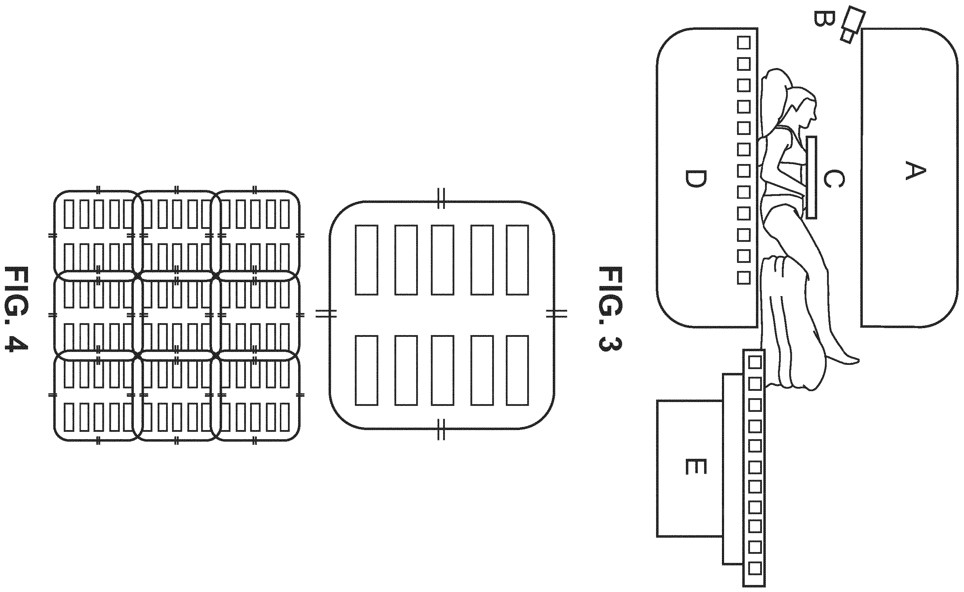

The medical imaging system and method of medical imaging are now described in more detail with respect to specific embodiments, where reference is made to Figs. 3-4.

Fig. 3 shows a detailed embodiment of a medical imaging system. In this specific embodiment the imaging acquisition unit is an MRI scanner, where the magnetic system is represented at "A". A camera represented at "B" acquires optical imagery of the patient. An array of RF transmitters (or transceivers), indicated as "D", is located in the patient support or mattress. A radar reflector is indicated at "C" (however "C" can represent RF coil receivers if a RF transmitter is used in the mattress for example). A table of the system is indicated at "E". Thus there can be a transceiver on one side of the patient, or a transmitter and receiver on the same side of the patient, or a transmitter and receiver on different sides of the patient, where for example the receiver could be placed on the patient or could be mounted fixedly adjacent to the magnet. The transmitter and receiver or transceiver form a radar apparatus, and this operates over tuneable frequencies.

The Radar apparatus emits radiation that is either bounced off the patient or passes through the patient to monitor the motion of the patient, from which trigger points for the MRI to acquire imagery can be determined. The monitored motion can be surface motion or even motion of some internal structure. What the radar apparatus 'sees' is largely dependent on the operating frequency. For a simple 1D motion signal, no image formation is necessary. Thus a single transmit-receive (radar) apparatus is sufficient. However, it can be desirable not just to determine a 1D signal but to observe several regions within the body that exhibit motion. For example to monitor the chest wall and belly in case of breathing based triggering, or parts of the heart and aorta in case of cardiac triggering. This is realized using an array of radar elements. However, the system shown in Fig. 3 is a development of that where a phased array is used for 'beam steering', where the phases between different emitted beam portions are delayed one to other, like a phase array aeronautical radar, to provide beam steering to interrogate exactly the required part of parts of the body. Thus, depending on the antenna design, the directivity of the radar beam can be tuned and the position for the trigger area can be selected/tuned. An area scan selecting the different directivities can be used to determine the best signal to noise for the best signal position, and helps to automatically do the positioning. This provides for a superior signal quality from which a trigger signal can be determined for triggering the MRI unit. However, the 1D single transmitter single receiver, as referred to above, can also be used to this effect.

Thus, depending on the frequency, the radar waves can be optimised to only 'see' the surface (or outmost few mm to cm) of the patient's body (microwave/THz-range) or can be optimised travel through the body entirely ('low' radio frequency), or only pass a certain way through the body suffering scattering and reflection on the way. Thus, transmitters can be placed below the patient bed and can also be placed above the patient.

Reflectors, can be used for example as stickers placed on top of the patient. Thus, radiation transmitted through the patient from a transmitter in the mattress can pass through the patient and reflect off the reflectors to provide an improved signal. Or a transmitter can be positioned above the patient to bounce off reflectors on top of the patient. Reflectors are not necessary as there is reflectance at the surface of the patient, and indeed when a transmitter and receiver are at fixed positions either side of the patient, motion information can still be obtained because the transmitted radar radiation suffers a temporal lag as it passes through the patient. For the MRI system RF-receive coils are already placed on top of the patient for MR-image acquisition. Thus, reflectors can be associated with the RF coils. Additionally, the transmitter or receiver or transceiver of the radar apparatus can be within these coils, or in the wall of the scanner bore. It is to be noted that for an efficient exam workflow and for patient comfort a placement of the radar apparatus in or beneath the patient table is convenient, because there is no additional weight on the patient, no additional space in the bore taken away for further equipment, and no cabling issues.

As detailed above, the radar apparatus operates at a correct frequency of radiation and emits radiation to interrogate the required parts of the patient. It is to be noted that this frequency is not the RF-frequency of the MRI unit used for MR-imaging, but is the frequency of radiation of the radar apparatus used to determine trigger points for the MRI unit. The radar apparatus can be fully stand-alone, but can also reuse some of the already present RF technology used for MR image generation. The selection of optimum monitoring frequencies by the radar apparatus, from which triggering of the MRI unit can be initiated, depend on patient size, position, and pose. This is where the camera or cameras are used in conjunction with the radar apparatus itself. This is because optical imagery acquired of the patient is used to augment the radar based data to determine the optimum operating frequencies, where for example preliminary data acquired by the radar apparatus can be used with the image data to determine the optimum frequencies for the radar apparatus from which trigger points can be determined. Thus, broadband radar reflectors, that reflect in the visible and in the RF region as discussed above, can for example be incorporated in a surface coil or applied as stickers on top of the patient, and this can help in generating optimum monitoring signals.

Returning to Fig. 3, as discussed above, reflectors such as stickers placed on the patient or incorporated into RF coils used as part of the MRI unit increase the reflectance of the surface of the person to the radiation emitted from the radar apparatus, whether using signal transmission from below and through the body, or having the transmitter and receiver or transceiver above the body and just "seeing" the top surface of the body. The reflectors are designed to take into account that they are present in the MRI unit during MR-imaging, and as such they need to be transparent for the RF-frequencies used for imaging (that is 64MHz for 1.5T systems and 127 MHz for 3T machines). Thus, the reflectors, such as large metallic patches, are split into smaller sub-patches in a in a certain way, such that no eddy currents are induced during the MR excitation RF-pulse (where up to several kW of RF-power is transmitted). Such eddy current mitigating design of features is known in the art, and is not discussed further here. It is to be noted that although an MRI unit is being discussed here, the discussed triggering system can be applied to other imaging modalities such as CT, PET, LINAC.

Continuing with Fig. 3, and returning to the specific embodiment of a MRI acquisition unit being used, as part of the MRI system RF-coils are used for signal reception only (which have to be resonant on the MR frequency during signal reception), and which are actively turned off during signal excitation. Otherwise these antennas would be damaged and would be of severe danger to the patient due to very high voltages that would be induced. As discussed above, the reflectors can also be incorporated within the RF coils in order to save space, and indeed the transmitter, receiver or transceiver can also be incorporated within the RF coils. The RF coils can be placed on the patient. Also, or alternatively a mattress with radar reflectors upon which the patient lies can be fixed on the surface of RF coils by stitching, using adhesives, with mechanical fasteners, or other means. However, similarly to the requirement to turn the RF coils off during activation of the MRI signal excitation, it has been found that for some of the RF frequencies of the radar system the RF coils, either turning off of the RF coils, via for example MEMS switches is required, or an active detuning can be necessary. Thus, local coils can be utilized that can already receive narrow band signals, and which are located outside the image band (pilot tone). Here the monitoring frequency of the radar apparatus can be close to the MR frequency. The radar apparatus uses broadband antennas (or different antennas for alternative frequencies) and the radar apparatus receives motion modulated (amplitude & phase) signals on selected frequencies optimal for motion detection, and where signal quality is boosted using radar reflectors on top on the patient. These could however be incorporated into an RF-coil element or array.

Fig. 4 shows an example of an RF coil element, with integrated radar reflector patches. Also shown is a coil array built up of several (in this case 12) of these coil elements. Such a configuration is also suitable for the receive coils for an MRI unit, but now there are integrated reflectors. The radar reflectors can be designed such that sufficient coverage with suitable reflectors is provided over a desired frequency range. As discussed above, the radar reflector patches are configured as broadband reflective devices such as broadband patch devices, and instead of being incorporated into an RF coil, such radar reflectors could also be applied on tight clothing or as adhesive stickers. Optimized positioning of the radar reflector stickers or mattress can be supported by cameras and and/or a software monitoring tool, and the radar reflectors can be equipped with optical markers. Additionally, slotted patches (or a larger number of small patches for that matter) are utilized in order not to interfere with the imaging process.

Regarding data processing, in a specific embodiment convolutional neural networks (CNN) / capsule networks are used to process the camera data and the radar data with this being fed to a recurrent neural network. The convolutional neural network / capsule network is of the feed forward network type to process the images efficiently. The recurrent neural network (RNN) is a class of artificial neural network, where connections between nodes form a directed graph along a sequence. This allows it to exhibit dynamic temporal behaviour for a time sequence. Long Short Term Memory or Gated Recurrent Units (GRU) type of RNNs are used, which can use their internal state (memory) to process sequences of inputs (here different frequencies). Attention is a memory access mechanism and this is utilized and takes into account input from several time steps to predict the required frequency of the radar apparatus. The ensemble of CNN, RNN and attention type of networks is suitable for selecting the optimum operating RF frequency for triggering signal generation. This makes them applicable to tasks such as unsegmented, connected motion recognition or camera motion recognition. However, other ways of using the camera imagery and radar data itself to determine the RF frequency of the radar apparatus, and then using the depth information to trigger the image acquisition unit such as a MRI unit or CT unit can be undertaken.

Thus in summary for a specific MRI embodiment:
- An array of local RF (radar) transceivers and antennas (phased array) of a radar apparatus are located in the MRI bore or the patient mattress. The selection of the operating frequency of the radar apparatus depends on patient size, position and pose which are detected by optical cameras along with preliminary radar data. These input values are utilized for finding the optimal frequency/frequencies for the application and target region (breathing / cardiac).
- Thus, a robust motion detection method is enabled, which uses a distinct frequency, multi frequency or broadband signal for the radar apparatus for motion detection.
- Monitoring signals are optimized by local broadband (radar-) reflectors, integrated into surface coils or realized as disposable stickers.
- An ensemble of neural networks such as convolutional, attention and recurrent networks is used to process the camera and preliminary radar data, select the optimum operating RF-frequency of the radar apparatus, and process the motion data of the patient and determine trigger signal generation for the MRI unit.

In another exemplary embodiment, a computer program or computer program element is provided that is characterized by being configured to execute the method steps of the method according to one of the preceding embodiments, on an appropriate system.

The computer program element might therefore be stored on a computer unit, which might also be part of an embodiment. This computing unit may be configured to perform or induce performing of the steps of the method described above. Moreover, it may be configured to operate the components of the above described apparatus and/or system. The computing unit can be configured to operate automatically and/or to execute the orders of a user. A computer program may be loaded into a working memory of a data processor. The data processor may thus be equipped to carry out the method according to one of the preceding embodiments.

This exemplary embodiment of the invention covers both, a computer program that right from the beginning uses the invention and computer program that by means of an update turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A medical imaging system (10), comprising:
- an image acquisition unit (20);
- a radar apparatus (30); and
- a processing unit (50);
wherein, the image acquisition unit is configured to acquire medical image data of a patient when positioned for medical image acquisition;
wherein, the radar apparatus comprises a transmitter (60) and receiver (70);
wherein, the radar apparatus is configured to operate over radio-wave and/or microwave and/or terahertz radiation frequencies to acquire depth information relating to the patient when positioned for medical image acquisition;
wherein, the processing unit is configured to determine at least one operational parameter for the radar apparatus comprising utilization of data acquired by the imaging system;
wherein, the processing unit is configured to operate the radar apparatus to acquire operational depth information relating to the patient comprising utilization of the at least one operational parameter; and
wherein, the processing unit is configured to control the image acquisition unit comprising utilization of the operational depth information.

2. System according to claim 1, wherein the processing unit is configured to operate the radar apparatus to acquire preliminary depth information relating to the patient when positioned for medical image acquisition, and wherein determination of the at least one operational parameter comprises utilization of the preliminary depth information.

3. System according to any of claims 1-2, wherein the system comprises at least one camera (40) configured to acquire optical image data of the patient when positioned for medical image acquisition, and wherein determination of the at least one operational parameter comprises utilization of the optical image data of the patient when positioned for medical image acquisition.

4. Medical imaging system according to any of claims 1-3, wherein the at least one operational parameter comprises an operational radiation frequency for the radar apparatus, and wherein the processing unit is configured to control the radar apparatus to operate at the operational radiation frequency to acquire the operational depth information relating to the patient.

5. Medical imaging system according to any of claims 1-4, wherein the at least one operational parameter comprises an indication to scan at least one specific region relative to a central axis of the radar apparatus, and wherein the processing unit is configured to control the radar apparatus to scan the at least one specific region to acquire the operational depth information relating to the patient.

6. Medical imaging system according to claim 5, wherein the transmitter of the radar apparatus comprises a plurality of transmitter elements.

7. Medical imaging apparatus according to claim 6, wherein the transmitter comprises a phased array transmitter, and wherein the processing unit is configured to control the radar apparatus to vary a direction of radiation with respect to the central axis.

8. Medical imaging system according to any of claims 1-7, wherein the processing unit is configured to utilize the operational depth information to trigger the image acquisition unit to acquire the medical image data.

9. Medical imaging system according to any of claims 1-8, wherein the transmitter and receiver of the radar apparatus are located on the same side of the patient when positioned for medical image acquisition.

10. Medical imaging system according to any of claims 1-8, wherein the transmitter and receiver of the radar apparatus are located on opposite sides of the patient when positioned for medical image acquisition.

11. Medical imaging system according to any of claims 1-10, wherein the system comprises at least one radiation reflector.

12. Medical imaging system according to claim 11, wherein the radiation reflector associated with the part of the image acquisition unit is comprised within or on a mattress of the image acquisition unit.

13. Medical imaging system according to any of claims 11-12, wherein the image acquisition unit is a Magnetic Resonance image acquisition unit, and wherein one or more radiation reflectors are comprised within one or more RF coil radiation receivers (72) of the image acquisition unit.

14. A method (100) of medical imaging with a medical imaging system, comprising:
a) positioning (110) a patient at least partially within an image acquisition unit of the medical imaging system;
b) acquiring (120) by the medical imaging system data of the patient when positioned at least partially within the image acquisition unit;
c) determining (130) by a processing unit of the medical imaging system at least one operational parameter for a radar apparatus of the medical imaging system comprising utilizing the optical image data, wherein the radar apparatus comprises a transmitter and receiver;
d) operating (140) by the processing unit the radar apparatus to acquire operational depth information relating to the patient when positioned at least partially within an image acquisition unit comprising utilizing the at least one operational parameter; and
e) controlling (150) by the processing unit the image acquisition unit to acquire medical image data of the patient comprising utilizing the operational depth information.

15. A computer program element for controlling a system according to any of claims 1-13, which when executed by a processor is configured to carry out the method of claim 14.
